Europäisches Patentamt

European Patent Office

Office européen des brevets

(11) Veröffentlichungsnummer : **0 154 303**
**B1**

(12) # EUROPÄISCHE PATENTSCHRIFT

(45) Veröffentlichungstag der Patentschrift :
26.04.89

(51) Int. Cl.⁴ : **A 61 K 7/42**

(21) Anmeldenummer : **85102256.6**

(22) Anmeldetag : **28.02.85**

(54) **Licht- und Sonnenschutzmittel.**

(30) Priorität : 08.03.84 DE 3408406

(43) Veröffentlichungstag der Anmeldung :
**11.09.85 Patentblatt 85/37**

(45) Bekanntmachung des Hinweises auf die Patenterteilung : **26.04.89 Patentblatt 89/17**

(84) Benannte Vertragsstaaten :
**AT BE CH DE FR GB IT LI NL SE**

(56) Entgegenhaltungen :
**CH—A— 387 228**
**DE—A— 3 233 388**
**FR—A— 1 203 888**
**PATENT ABSTRACTS OF JAPAN, unexamined applications, Sektion C, vol. 7, No. 100, 28. April 1983; THE PATENT OFFICE JAPANESE GOVERNMENT, Seite 60C164**

(73) Patentinhaber : **Beiersdorf Aktiengesellschaft**
**Unnastrasse 48**
**D-2000 Hamburg 20 (DE)**

(72) Erfinder : **Henne, Wolfgang, Dr. Dipl-Chem.**
**Astweg 8**
**D-2000 Hamburg 54 (DE)**
Erfinder : **Hoppe, Udo, Dr. Dipl.-Chem**
**Lottbeker Weg 7**
**D-2000 Hamburg 65 (DE)**

**Beschreibung**

Die Erfindung betrifft Licht- und Sonnenschutzmittel zur Anwendung auf der menschlichen Haut, die neben üblichen UV-Filtersubstanzen zur Erhöhung der Lichtschutzwirkung Sericin enthalten.

Bekanntlich erzeugen Lichtstrahlen des Wellenlängenbereichs von etwa 280 bis 320 nm (sog. UV-B-Strahlung) bei hellhäutigen Menschen unerwünschte Hautschädigungen (Sonnenbrand, Erythem), wenn ihre Haut einer starken und langfristigen Einwirkung der Sonne oder einer künstlichen Lichtquelle ausgesetzt wird sowie die langanhaltende sog. indirekte oder verzögerte Bräunung. Die tiefer in die Haut eindringenden Ultraviolettstrahlen im Wellenlängenbereich von etwa 320 bis 400 nm (sog. UV-A-Strahlung) verursachen dagegen die sog. direkte Bräunung der Haut, die allerdings nur von kurzer Dauer ist.

Zweck von kosmetischen Licht- und Sonnenschutzmitteln ist es daher, die Haut so vollkommen wie möglich vor den ungünstigen Einwirkungen des Sonnenlichts oder einer künstlichen UV-Lichtquelle zu schützen unter kleinstmöglicher Beeinträchtigung der günstigen Effekte, besonders des Bräunens.

Man muß dafür sorgen, daß die Stimulation der Bräunung mit suberythemalen Bestrahlungsdosen ohne die Auslösung eines Sonnenbrandes (Erythems) erreicht wird.

Dies wird bewirkt durch die Verwendung geeigneter Filtersubstanzen für den UV-B-Bereich, die durch Herausfiltern (Absorbieren) der für die Erythembildung verantwortlichen Strahlen die Erythembildung verhindern, ohne jedoch die Pigmentbildung in der Haut, die die Ursache für die Bräunung darstellt, zu beeinträchtigen.

Kosmetische Licht- und Sonnenschutzpräparate kommen in Form von wässrigen, wässrig-alkoholischen oder öligen Lösungen, in Emulsionsform als Cremes oder Salben, als Gele, als Lotions oder als aus Aerosolbehältern versprühbare Zusammensetzungen in den Handel. Der nach dem Auftragen eines solchen Präparats auf der Hautoberfläche verbleibende, die UV-Filtersubstanz enthaltende Film absorbiert bei der Einwirkung von Sonnenlicht oder künstlichem UV-Licht zumindest den größten Teil der Strahlen im UV-B-Bereich, während die Strahlen höherer Wellenlängen im UV-A-Bereich mehr oder weniger ungehindert durchgelassen werden. Dabei ist die Intensität der Lichtschutzwirkung von der verwendeten Grundlage und bei gleicher Grundlage von der Wirkstoffkonzentration abhängig.

Zur Verwendung als UV-Filtersubstanzen in Licht- und Sonnenschutzmitteln zur Anwendung auf der menschlichen Haut wurden bereits zahlreiche Verbindungen vorgeschlagen, die den verschiedensten organisch-chemischen Verbindungsklassen angehören. Zu den bekanntesten, für diesen Zweck eingesetzten Verbindungen (UV-Absorbern) gehören Zimtsäurederivate, Benzophenonderivate, 2-Phenylbenzimidazolderivate, Kampferderivate, p-Aminobenzoesäurederivate sowie Salicylsäurederivate. Die Wirksamkeit jeder Lichtschutzsubstanz ist bei gegebener Konzentration im Präparat u.a. abhängig von der Schichtdicke des Auftrags aus der Hautoberfläche, und diese ist wiederum abhängig von der Viskosität des Licht- oder Sonnenschutzmittels.

Bei der Auswahl einer zur Herstellung eines kosmetischen Licht- oder Sonnenschutzmittels geeigneten UV-Filtersubstanz aus der Vielzahl der für diesen Zweck vorgeschlagenen Substanzen, die je nach dem Extinktionskoeffizienten der gewählten Substanz im UV-B-Bereich bei ca. 308 nm (Bereich maxim. Erythembildung) und der bei der Anwendung auf der Haut erzielbaren Schichtdicke im allgemeinen in einer Menge von 0,1 bis 15 Gewichts-%, vorzugsweise von 1 bis 10 Gewichts.-%, bezogen auf die Gesamtzusammensetzung des Mittels, in dem Licht- und Sonnenschutzmittel eingesetzt werden, müssen eine Reihe von Anforderungen berücksichtigt werden, die häufig nur unter Schwierigkeiten in der gewünschten Weise miteinander kombiniert werden können.

1. Die UV-Filtersubstanz muß haut- und schleimhautverträglich und darf nicht toxisch sein, darf daher auf die Haut weder eine irritierende, noch eine sensibilisierende Wirkung entfalten. Dies ist insofern von besonderer Bedeutung, als Licht- und Sonnenschutzpräparate oft regelmäßig auf großen Teilen der Körperoberfläche aufgetragen werden, deren Widerstandskraft häufig durch vorherige Einwirkung von Sonne, Kälte, Wind und Wasser erheblich herabgesetzt ist.

2. Neben einer hohen Filterwirksamkeit im erythembildenden UV-B-Bereich soll die Substanz eine verhältnismäßig hohe Durchlässigkeit im UV-A-Bereich aufweisen (selektive Absorption). Das Absorptionsmaximum muß im Maximum der Erythemwirkung der Globalstrahlung bei 307 bis 308 nm liegen.

3. Die UV-Filtersubstanz muß licht- und thermostabil sein sowie chemisch inert, d.h. : sie darf unter der Einwirkung von UV-Strahlen, Wasser oder Schweiß keine chemischen Veränderungen erfahren, durch die ihre Schutzwirkung herabgesetzt wird oder eine Reizwirkung auf die Haut entstehen könnte.

4. Die Substanz darf von der Haut weder resorbiert werden, noch sich innerhalb weniger Stunden verflüchtigen.

5. Die Substanz muß geruchlos sein und darf die Haut nicht verfärben.

6. Sie muß eine ausreichende Löslichkeit im Fertigprodukt aufweisen, mit den gebräuchlichen kosmetischen Grund- und Zusatzstoffen verträglich und befähigt sein, als Bestandteil eines Licht- oder Sonnenschutzmittels einen dünnen, zusammenhängenden und dauerhaften Schutzfilm auf der Haut zu bilden.

Die Aufgabe der Erfindung bestand darin, Substanzen aufzufinden, die schon bei Zusatz kleiner Anteile zu UV-Filtersubstanzen enthaltenden kosmetischen Licht- und Sonnenschutzmitteln eine Erhöhung der Lichtschutzwirkung hervorrufen (Anhebung der Erythemschwellenzeit) bzw. bei Zusatz solcher Substanzen eine Verringerung der zur Erreichung eines definierten Lichtschutzes notwendigen UV-Filtermenge ermöglichen. Dies ist insofern von erheblicher praktischer Bedeutung, als viele wertvolle UV-Filtersubstanzen (UV-Absorber) in vielen kosmetischen Grundlagen häufig nicht die erwünschte, zur Erzielung eines definierten Lichtschutzes erforderliche Löslichkeit aufweisen. Hinzu kommt, daß der Einsatz von preisgünstigen, die Lichtschutzwirkung erhöhenden Substanzen in kosmetischen Licht- und Sonnenschutzmitteln es ermöglicht, zur Erzielung der gleichen Lichtschutzwirkung einen Teil der häufig recht teuren UV-Filtersubstanzen durch solche preisgünstigeren Substanzen ersetzen zu könner. Hierbei ist zu beachten, daß eine weitere Erhöhung einer UV-Filtersubstanz bei mehrprozentiger Einsatzkonzentration schließlich kaum noch eine Erhöhung des Erythemschutzes bewirkt.

Es wurde überraschenderweise gefunden, daß Sericin in kleinen Anteilen kosmetischen Licht- und Sonnenschutzmitteln zugesetzt, die übliche UV-Filtersubstanzen in einer für derartige Präparate üblichen Menge enthalten, eine deutliche Erhöhung der Lichtschutzwirkung zu bewirken vermag. Dies konnte im Hinblick darauf, daß Sericin selbst nur eine vernachlässigbar geringe Filterwirkung im Bereich unterhalb des UV-A-Bereichs im UV-B-Bereich aufweist, nicht erwartet werden und war daher für den Fachmann nicht vorhersehbar. Dieses Ergebnis der Erhöhung der Lichtschutzwirkung von UV-Filtersubstanzen enthaltenden Licht- und Sonnenschutzmitteln durch den Zusatz von Sericin konnte anhand von klinischen Untersuchungen (in-vivo-Vergleichsversuchen), die mit jeweils 20 Probanden durchgeführt wurden, in vollem Umfang bestätigt werden.

Gegenstand der Erfindung sind somit Licht- und Sonnenschutzmittel für die menschliche Haut auf Basis üblicher UV-Filtersubstanzen, die dadurch gekennzeichnet sind, daß sie zusätzlich Sericin enthalten.

Als Sericin bezeichnet man das Schutzprotein, das das Fibrion des vom Seidenspinner (Bombyx mori) in Form eines Doppelfadens ausgeschiedenen (gesponnenen) fadenartigen Gebildes mantelförmig umhüllt. Wird eine geeignete Anzahl dieser umhüllten Doppelfäden umeinander verdreht, so ergibt sich ein Rohseidenfaden, der anschließend zu einem Rohseidengewebe verwebt werden kann. Je nach Dicke dieser Doppelfäden, die von Jahreszeit zu Jahreszeit schwankt, werden 7 bis 13 Doppelfäden für einen einzigen Rohseidenfaden benötigt. Das hieraus resultierende Rohseidengewebe kann bereits als solches für verschiedene Zwecke eingesetzt werden. Um jedoch weichere Seidengewebe zu erhalten, wird anschließend das Sericin, das 19 bis 28 Gewichts-% der Rohseide ausmacht und das hinsichtlich der Zusammensetzung und der Eigenschaften erhebliche Unterschiede gegenüber dem Seidenfibroin aufweist, durch Behandlung mit einer Seifenlösung, insbesondere mit sog. « Marseiller Seife », oder der wässrigen Lösung eines geeigneten Netzmittels bzw. durch Einwirkung geeigneter Enzyme vom Fibroin getrennt.

Für den erfindungsgemäßen Zweck kann das auf diese Weise gewonnene Sericin in der durch Sprühtrocknung aus derartigen Lösungen unter Vakuumbedingungen erhältlichen feinpulvrigen Form verwendet werden, vorzugsweise jedoch in Form der stark verdünnten wässrigen Lösung des Extrakts der Behandlung der Rohseide mit « Marseiller Seife » (etwa 2 %ige Lösungen). Als « Marseiller Seife » wird eine harte, weiße, gelbliche oder grünliche Natronkernseife auf extrahiertem Olivenöl bezeichnet, die zur Herstellung von Toiletten- und Textilseifen dient.

Die Menge an Sericin in den Licht- und Sonnenschutzmitteln gemäß der Erfindung kann 0,05 bis 5,0 Gewichts-%, vorzugsweise 0,25 bis 1,5 Gewichts-%, und die Menge an UV-Filtersubstanzen 0,5 bis 15 Gewichts-%, vorzugsweise 1 bis 10 Gewichts-%, betragen, jeweils bezogen auf die Gesamt-Zusammensetzung des Mittels. Dabei kann anstelle einer UV-Filtersubstanz auch eine Kombination mehrerer UV-Filtersubstanzen unter Einbeziehung von UV-A-Filtersubstanzen verwendet werden, die verschiedenen organischchemischen Verbindungsklassen angehören können und für die je nach Art der kosmetischen Grundlage sowohl öllösliche aus auch wasserlösliche Typen oder eine Mischung aus diesen ausgewählt werden können.

Ohne jedwede Beschränkung auf die genannten Verbindungstypen von UV-Filtersubstanzen kann Sericin für den erfindungsgemäßen Zweck vorteilhaft kombiniert werden mit einer UV-Filtersubstanz vom Triazin-Typ, z. B. 2,4,6-Trianilino-(p-carbo-2-äthylhexyl-1-oxi)-s-triazin, vom Zimtsäureester-Typ, z. B. p-Methoxy-äthylhexyl-zimtsäure-ester, vom Benzylidenkampfer-Typ, z. B. 3-(4-Methylbenzyliden)-D,L-kampfer oder vom 2-Phenylbenzimidazol-Typ, z. B. 2-Phenylbenzimidazol-5-sulfonsäure, insbesondere in Form ihrer Natriumsalze, Mono- oder Triäthanolammoniumsalze.

Die erfindungsgemäßen Licht- und Sonnenschutzmittel können in Form der bekannten Sonnenschutzmittel z. B. in Form von wasserfreien Präparaten (als flüssige Lichtschutzöle), in Emulsionsform als nichtfettende O/W-Emulsionen, halbfette Mischemulsionen oder fettreiche W/O-Emulsionen (Cremes oder milchartige Zubereitungen), als Lotionen wie auch als fettfreie alkoholhaltige Präparate sowie in Form von aus Aerosoldosen versprühbare Zusammensetzungen vorliegen.

Die Licht- und Sonnenschutzmittel gemäß der Erfindung können in Anpassung an die jeweils gewünschte Zubereitungsform in derartigen Mitteln übliche kosmetische Grund- und Zusatzstoffe in ebenfalls üblichen Mengenverhältnissen enthalten, wie pflanzliche oder tierische Fette und Wachse, z. B. Olivenöl, Erdnuß- oder Sesamöl, Wollwachsderivate, Fettsäuren und Fettsäureester, z. B. Stearinsäure, Palmitinsäure, Ölsäure, Glycerinmono- oder -distearat, Glycerinmonoleat, Isopropylmyristat, Diisopropyladipat, feste und flüssige Kohlenwasserstoffe, z. B. Paraffin, Vaseline oder Paraffinöl, Alkohole, z. B.

Äthyl-, Isopropyl- Cetyl-, Stearyl- oder Palmitylalkohol, mehrwertige Alkohole, die als Feuchthaltemittel dienen, z. B. Glykole, Glycerin, Sorbit, sowie insbesondere Emulgatoren der Systeme Öl-in-Wasser (O/W) und Wasser-in-Öl (W/O), wofür die handelsüblichen Typen, insbesondere die nicht-ionogenen und anionaktiven Typen, in Betracht kommen. Weiterhin können die Licht- und Sonnenschutzmittel zur Verbesserung der Streichbarkeit und Erzielung einer erhöhten Schichtdicke beim Auftrag auf die Haut Verdickungsmittel, wie Methyl-, Äthyl- oder Carboxy-methylcellulose, Polyacrylsäure, Alginate, Traganth, Agar-Agar oder Gelatine, enthalten.

Ferner können in die erfindungsgemäßen Licht- und Sonnenschutzmittel eingearbeitet werden : Geruchsstoffe, wie Lavendelöl, Nelkenöl, Citronenöl, oder Parfüm-Kompositionen, physiologisch unbedenkliche Farbstoffe, Konservierungsmittel, wie der Methyl- oder der n-Propylester der p-Hydroxybenzoesäure, Antioxydationsmittel, wie z. B. Butylhydroxytoluol oder Butylhydroxyanisol, sowie gegebenenfalls Insekten-Abwehrmittel, wie 3-(N-n-butyl-N-acetyl)-amino-propionsäureäthylester, m-Toluylsäure-N,N-diäthylamid oder 2-Äthyl-1,3-hexandiol.

Die jeweils einzusetzende Menge an diesen vorgenannten Zusatzstoffen kann in Abhängigkeit von der Art des jeweiligen Produktes vom Fachmann durch einfaches Ausprobieren leicht ermittelt werden. Sie beträgt für die Geruchsstoffe (Parfüm-Kompositionen), jeweils bezogen auf die Gesamt-Zusammensetzung des Mittels, normalerweise 0,1 bis 1,0 Gewichts-%, für Konservierungsmittel etwa 0,2 bis 0,5 Gew.-% und für Antioxydantien etwa 0,01 bis 0,05 Gew.-%.

Treibmittel für aus Aerosoldosen versprühbare Zusammensetzungen sind insbesondere chlorfluorierte Kohlenwasserstoffe, wie Dichlordifluormethan und Tetrafluordichloräthan, Propan und Butan oder deren Gemische.

Vorzugsweise werden die Licht- und Sonnenschutzmittel gemäß der Erfindung auf einen neutralen bis schwach sauren pH-Wert eingestellt (pH : 4-7).

Die Herstellung der Licht- und Sonnenschutzpräparate erfolgt in üblicher Weise, indem die die Fettphase bildenden Bestandteile und die die Wasserphase bildenden Bestandteile unabhängig voneinander vermischt und getrennt auf etwa 75 °C erwärmt werden. Anschließend wird bei dieser Temperatur die Wasserphase in die als Schmelze vorliegende Fettphase eingerührt, die so erhaltene Mischung haltgerührt und vor oder nach Zugabe der Geruchsstoffe bei etwa 35 °C in einem üblichen Homogenisator homogenisiert.

Die folgenden Beispiele sollen die Erfindung näher erläutern, ohne diese darauf zu begrenzen :

Beispiel 1

Sonnenschutz-Lotion

|  | Gew.-% |
|---|---|
| a) Stearinsäure | 1,50 |
| Glycerinmonostearat | 1,00 |
| Cetylalkohol | 0,40 |
| 2,4,6-Trianilino-(p-carbo-2-äthylhexyl-1-oxi)-s-triazin | 4,00 |
| Diisopropyladipat | 5,00 |
| b) Triäthanolamin | 0,75 |
| Glycerin | 4,00 |
| Polyacrylsäure | 0,10 |
| Sericinlösung (2 %ig) | 50,00 |
| Wasser, entmineralisiert | 32,50 |
| Konservierungsmittel | 0,20 |
| c) Parfum | 0,55 |
|  | 100,00 |

Herstellung : Man erwärmt die Phasen a) und b) getrennt voneinander auf etwa 75 °C und rührt bei dieser Temperatur Phase b) in Phase a) ein, kühlt das so erhaltene Gemisch unter Rühren auf etwa 35 °C ab, gibt c) hinzu und homogenisiert.

Beispiel 2

Sonnenschutz-Lotion

|  | Gew.-% |
|---|---|
| a) Cetylalkohol | 0,50 |

| | |
|---|---|
| Gem. aus Cetylstearylalkohol und Natrium-Cetylstearylsulfat im Verh. 90 :10 | 2,00 |
| Glycerinmono-/-distearat | 1,00 |
| Paraffinöl | 6,00 |
| p-Methoxy-äthylhexyl-zimtsäureester | 5,00 |
| b) Triäthanolamin | 0,17 |
| Polyacrylsäure | 0,20 |
| Sericinlösung (2 %ig) | 50,00 |
| Wasser, entmineralisiert | 34,43 |
| Konservierungsmittel | 0,50 |
| c) Parfum | 0,20 |
| | 100,00 |

Herstellung : wie im Beispiel 1 beschrieben.

## Beispiel 3

### Sonnenschutz-Creme

| | Gew.-% |
|---|---|
| a) Cetylalkohol | 3,50 |
| Gem. aus Glycerinmonodistearat mit Polyglykolfettalkoholäthern (« Teginacid ») | 3,00 |
| Paraffinöl | 12,00 |
| p-Methoxyzimtsäure-äthylhexylester | 1,00 |
| 3-(4-Methylbenzyliden)-D,L-kampfer | 0,50 |
| b) Natriumsalz der 2-Phenylbenzimidazol-5-sulfonsäure | 1,00 |
| Natrium-Cetylstearylsulfat | 2,00 |
| Natriumsalz von Polyacrylsäure | 0,24 |
| Sericinlösung (2 %ig) | 25,00 |
| Wasser, entmineralisiert | 50,96 |
| Konservierungsmittel | 0,50 |
| c) Parfum | 0,30 |
| | 100,00 |

Herstellung : wie im Beispiel 1 beschrieben.

## Patentansprüche

1. Licht- und Sonnenschutzmittel für die menschliche Haut auf Basis üblicher UV-Filtersubstanzen, dadurch gekennzeichnet, daß sie zusätzlich Sericin enthalten.

2. Licht- und Sonnenschutzmittel nach Anspruch 1, dadurch gekennzeichnet, daß sie Sericin in einer Menge von 0,05 bis 5,0 Gewichts-%, vorzugsweise 0,25 bis 1,5 Gewichts-%, und die UV-Filtersubstanzen in einer Menge von 0,5 bis 15 Gewichts-%, vorzugsweise von 1 bis 10 Gewichts-%, jeweils bezogen auf die Gesamt-Zusammensetzung des Mittels, enthalten.

3. Licht- und Sonnenschutzmittel nach den Ansprüchen 1 oder 2, dadurch gekennzeichnet, daß sie Sericin in Kombination mit mindestens einer UV-Filtersubstanz vom Triazin-Typ, vom Zimtsäureester-Typ, vom Benzylidenkampfer-Typ oder vom 2-Phenylbenzimidazol-Typ oder deren Salze enthalten.

## Claims

1. Light- and sunscreens for the human skin based on customary UV filter substances, characterized in that they additionally contain sericin.

2. Light- and sunscreens according to claim 1, characterized in that they contain sericin in an amount of from 0.05 to 5.0 % by weight, preferably 0.25 to 1.5 % by weight, and the UV filter substances in an amount of from 0.5 to 15 % by weight, preferably 1 to 10 % by weight, in each case relative to the total composition of the agent.

3. Light- and sunscreens according to claims 1 or 2, characterized in that they contain sericin in combination with at least one UV filter substance of the triazine type, of the cinnamic acid ester type, of the benzylidene-camphor type or of the 2-phenylbenzimidazole type or their salts.

## Revendications

1. Agents protecteurs contre la lumière et le soleil pour la peau humaine à base de substances habituelles filtrant les UV, caractérisés en ce qu'ils contiennent en outre de la séricine.

2. Agents protecteurs contre la lumière et le soleil suivant la revendication 1, caractérisés en ce qu'ils contiennent la séricine en une quantité de 0,05 à 5,0 % en poids et de préférence de 0,25 à 1,5 % en poids, et les substances filtrant les UV en une quantité de 0,5 à 15 % en poids et de préférence de 1 à 10 % en poids, chacune sur la base de la composition totale de l'agent.

3. Agents protecteurs contre la lumière et le soleil suivant la revendication 1 ou 2, caractérisés en ce qu'ils contiennent la séricine en combinaison avec au moins une substance filtrant les UV du type de la triazine, du type des esters d'acide cinnamique, du type du benzylidène-camphre ou du type du 2-phénylbenzimidazole ou de leurs sels.